**Europäisches Patentamt**

(19) **European Patent Office**

**Office européen des brevets**

(11) Publication number : **0 506 790 B1**

## EUROPEAN PATENT SPECIFICATION

(45) Date of publication of patent specification :
08.09.93 Bulletin 93/36

(51) Int. Cl.$^5$ : **C12P 19/18**

(21) Application number : **91901707.9**

(22) Date of filing : **21.12.90**

(86) International application number :
**PCT/DK90/00338**

(87) International publication number :
**WO 91/09962 11.07.91 Gazette 91/15**

(54) **A METHOD FOR ENZYMATICALLY CONVERTING STARCH INTO CYCLODEXTRINS.**

(30) Priority : **22.12.89 US 455188**

(43) Date of publication of application :
**07.10.92 Bulletin 92/41**

(45) Publication of the grant of the patent :
**08.09.93 Bulletin 93/36**

(84) Designated Contracting States :
**AT BE CH DE DK ES FR GB GR IT LI LU NL SE**

(56) References cited :
**WO-A-88/08031**
**WO-A-89/01043**
**WO-A-89/01044**
**WO-A-89/03421**
**Chemical Abstracts, vol. 111, no. 17, 23
October 1989, (Columbus, Ohio, US), see ab-
stract 152178N**
**Patent Abstracts of Japan, vol. 9, no 271, C311**

(56) References cited :
**Chemical Abstracts, vol. 111, no. 25, 18 De-
cember 1989 (Columbus, Ohio, US), C.P.S.
Yang et al.: "Study of cyclodextrin production
using caclodextrin glycosyltransferase im-
mobilized on chitosan", p. 612, abstract
230637c**
**Chemical Abstracts, vol. 104, no. 1, 6 Jan. 1986
(Columbus, Ohio, US), p. 428, abstract 4653d**
**Chemical Abstracts, vol. 109, no. 5, 1 August
1988, (Columbus, Ohio, US), E.K.C. Yu et al.:
"Specific alpha-cyclodextrin production by a
novel thermostable cyclodextrin glycosyl-
transferase", p. 503, abstract 36564v**
**Chemical Abstracts, vol. 111, no. 3, 17 July
1989, (Columbus, Ohio, US), p. 502, abstract
222 Old**

(73) Proprietor : **NOVO NORDISK A/S**
**Novo Allé**
**DK-2880 Bagsvaerd (DK)**

(72) Inventor : **STARNES, Robert, L.**
**48 Rio Del Oro Lane**
**Sacramento, CA 95825 (US)**

(74) Representative : **Knudsen, Sten Lottrup et al**
**c/o Novo Nordisk A/S, Patent Department,**
**Novo Allé**
**DK-2880 Bagsvaerd (DK)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

This invention relates to a process for producing cyclodextrin, said process comprising enzymatic treatment of a liquefied starch solution with cyclodextrin glycosyl transferase (CGTase) and thereafter recovery of a cyclodextrin product from the reaction mixture,

### BACKGROUND ART

Cyclodextrins, also known as Schardinger dextrins, are starch-derived cyclic compounds containing six, seven, or eight glucopyranose residues linked by alpha-1,4 bonds being termed α-, β-, or γ-cyclodextrin. The cyclic nature of these compounds produces a cavity which is predominantly hydrophobic enabling the formation of inclusion complexes with a number of compounds. This complexation ability is of particular interest to the food, agrochemical, cosmetic, and pharmaceutical industries for: taste masking, stabilization, increasing solubility, powdering, colour masking, and emulsification, to name a few possible uses.

A starch solution can be degraded into cyclodextrins by enzymes known as cyclodextrin glycosyl transferases (1,4-alpha-D-glucan 4-alpha-D-(1,4-alpha-D-glucano)-transferase, E.C. 2.4.1.19), hereinafter termed cyclodextrin glycosyl transferase or CGTase. The CGTase enzymes degrade the liquefied starch by catalyzing cyclization and disproportionation reactions.

Thus, the enzymatic cyclization reaction produces cyclodextrins, such being cyclic molecules comprised of six, seven, or eight alpha-D-glucopyranose residues linked by alpha-1,4-bonds. They are known as α-, β-, or γ-cyclodextrins depending on the number of glucose residues, 6, 7 or 8, respectively. These cyclized molecules have neither a non-reducing nor reducing end-group.

Importantly, reactions catalyzed by CGTase which cleave the starch molecule can generate a desirable viscosity reduction in liquefied starch solutions by lowering the average molecular weight of the dextrin in the solution (without, in the instance of the CGTase, generating reducing sugars). The CGTase enzymes previously known to the art were produced by such microorganisms as *Bacillus macerans*, *Bacillus circulans*, *Bacillus stearothermophilus*, *Bacillus megaterium*, *Bacillus ohbensis*, alkalophilic *Bacillus sp.*, *Micrococcus luteus*, *Micrococcus varians*, and *Klebsiella pneumoniae*. Unfortunately, none of the CGTase enzymes produced by these microorganisms seem to be sufficiently thermostable for use in industrial-scale for a combination of starch liquefaction and cyclodextrin production.

Recently, WO 89/03421 has described thermostable, aciduric CGTase from obligate anaerobic bacteria belonging to the genus *Thermoanaerobacter* and its use for production of cyclodextrin at 80-90°C. However, in all likelihood recombinant DNA manipulation will be required to produce the *Thermoanaerobacter* enzyme in commercial quantities, and the availability of alternative sources for thermostable, aciduric CGTase is therefore desirable.

### SUMMARY OF THE INVENTION

We have now discovered thermostable CGTase enzymes produced by strains of the thermophilic obligate anaerobic microorganisms *Clostridium thermoamylolyticum* ATCC 39,252 and *Clostridium thermohydrosulfuricum* ATCC 53,016. The CGTase enzymes are usable at pH 5.0-5.5 and 105°C.

US 4,578,352 and US 4,613,570 describe aciduric alpha-amylase enzymes from these two strains. Unfortunately, the strains produce the CGTase enzymes in such low yield that the inventors hereof are unable to establish for certain whether the CGTase enzymes of this invention are the same enzymes as reported in said two US patents. However, use of enzymes from these strains in production of cyclodextrin is novel and could not be predicted.

A number of thermophilic anaerobic microorganisms have been investigated in the prior art for unique, thermostable enzymes. Notably, species of the genus *Clostridium* have been the focus of attention including *Clostridium thermoamylolyticum*, *C. thermohydrosulfuricum*, *C. thermocellum*, *C. thermosaccharolyticum*, *C. thermoaceticum*, *C. thermosulfurogenes*, *C. stercorarium*, and *C. fervidus*. However, none of the *Clostridium* strains have been reported to produce a thermostable cyclodextrin glycosyl transferase, probably because CGTases were not being sought.

Accordingly, the invention provides an enzymatic process for converting a pre-liquefied starch into cyclodextrins with a Clostridium species CGTase enzyme followed by recovery of cyclodextrin.

A further aspect of this invention provides a process for converting solid starch into cyclodextrins which comprises liquefying the starch with the CGTase enzyme under typical starch liquefaction conditions which, for example, may be under the time and temperature liquefaction conditions used in the isosyrup industry followed by enzymatic conversion of the liquefied starch with the CGTase enzyme and recovery of cyclodextrin.

Preferred conditions for the enzymatic conversion to cyclodextrin are pH 5.0-5.5 at above about 60°C, preferably at reaction temperatures exceeding about 70°C, and more preferably exceeding about 85°C. Actually, conversion reaction temperatures exceeding about 70°C may be needed, if the CGTase enzyme has not been purified so as to inactivate any glucoamylase activity that may be present (see Example 3). Due to enzyme stability the reaction temperature is preferably below about 90°C. Treatment times not exceeding about 24 hours are preferred.

In a preferred mode of the invention, the process involves liquefaction of a starch slurry with the CGTase enzyme at about pH 5.0 at an initial treatment temperature exceeding about 100°C followed by conversion of the liquefied starch to cyclodextrins by maintaining the liquefied starch at about 80°-90°C for up to about 24 hours in a hold step, desirably without pH adjustment, and desirably without redosing with enzyme.

Suitable liquefaction conditions are about 1-60 minutes at about 100-115°C, preferably followed by holding for about 50 minutes to 4 hours at about 80-100°C. A continuous process is preferred, and the heating is most preferably by jet-cooking.

A dosage level of 2-5 Phadebas U (see below) per gram starch DS (dry substance) is suitable for liquefaction and enzymatic conversion according to the invention. The starch concentration will usually be in the range 15-40% DS (w/w% dry substance), most often 25-35% DS. A calcium salt may be added to a concentration of 20-100 ppm $Ca^{++}$.

The starch used in practice of this invention may be obtained from many different vegetable crop sources including, for example, corn, waxy maize, wheat, sorghum, potato, tapioca, rice, and sago. In addition to such raw forms of the starches, starch itself and modified forms of starch produced from treatment of the starch with enzymes, acids, alkalies, etc. can be used as substrates. The cyclodextrinization reaction can be performed on liquefied starch at DS concentrations ranging from 1 % to 40% DS, but a 25% DS solids solution is preferred. The cyclodextrin products may be recovered from the reaction solution according to known art practices.

The CGTase enzyme may be produced by cultivating a CGTase-producing *Clostridium* species strain, preferably ATCC 39.252 or ATCC 53,016 or a mutant or variant thereof under anaerobic conditions, or by cultivating (aerobically) a transformant containing the appropriate genetic information from ATCC 39,252 or ATCC 53,016 and, thereafter recovering the CGTase enzyme from the culture medium.

The process of this invention has a number of advantages. First, the starch does not need to be preliquefied, e.g., with an alpha-amylase to solubilize the starch. Second, the CGTase is sufficiently stable at above 60°C to be used at such higher temperatures, avoiding thereby possible microbial contamination. Third, the conversion of starch to cyclodextrins does not require extended reaction times before reasonable yields are achieved.

## BRIEF DESCRIPTION OF DRAWINGS

For further understanding of this invention, the detailed description thereof which follows is made with reference to the attached drawings wherein:

Figure 1 is a plot of relative activity versus temperature for the CGTase;

Figure 2 is a plot of relative activity versus pH for the CGTase;

Figure 3 is an HPLC plot showing the action pattern of CGTase from *Clostridium thermoamylolyticum* ATCC 39,252 at pH 5.5 on 35% DS corn starch.

Figure 4 is an HPLC plot of the action pattern of CGTase from *Clostridium thermohydrosulfuricum* ATCC 53,016 at pH 5.0 on 35% DS corn starch.

Figure 5 is an HPLC plot showing the action pattern of CGTase from *Clostridium thermoamylolyticum* ATCC 39,252 on a pre-liquefied corn starch (DE 33).

Figure 6 is an HPLC plot showing the action pattern of CGTase from *Clostridium thermohydrosulfuricum* ATCC 53,016 on a pre-liquefied corn starch (DE 68).

## DETAILED DESCRIPTION OF THE INVENTION

### Microorganism source

As has already been pointed out the CGTases herein exemplified are produced by the thermophilic obligate anaerobic bacteria *C. thermoamylolyticum* ATCC 39,252 and *C. thermohydrosulfuricum* ATCC 53,016. The CGTases are extracellular enzymes. Both of these strains were known to the art prior to this invention, having been reported as source microorganisms for an aciduric alpha-amylase.

U.S. Patent 4,578,352 describes the strain ATCC 39,252 and production of an enzyme identified as an aciduric alpha-amylase therewith. As has been pointed out above, the inventor hereof has been unable to ascertain if the aciduric alpha-amylase composition produced by the patentees of 4,578,352 contained CGTase.

U.S. Patent 4,613,570 describes the strain ATCC 53,016 and production of an aciduric alpha-amylase therewith. As has been pointed out above, the inventor hereof has been unable to ascertain if the aciduric alpha-amylase composition produced by the patentees of 4,613,570 contained CGTase.

Production of Cyclodextrin Glycosyl Transferase

The microorganisms were cultured and maintained using standard anaerobic methodology. Production of the CGTase was achieved by culturing the microorganisms in a pre.reduced liquid medium under argon comprised of the following components in grams per liter: Maltrin M-100, 8.0; $KH_2PO_4$, 1.5; $Na_2HPO_4\cdot12H_2O$, 4.2; $NH_4Cl$, 0.5; $MgCl_2\cdot6H_2O$, 0.18, yeast extract, 2.0; $Na_2S$, 0.5; cysteine-HCl, 0.5; resazurin (redox indicator), 2 ng; and trace metals 5.0 ml. The trace metal solution was comprised of the following components in grams per liter: $FeCl_3\cdot6H_2O$, 5.40; $ZnSO_4\cdot7H_2O$, 1.45; $MnCl_2\cdot4H_2O$, 1.00; $CuSO_4\cdot5H_2O$, 0.25; and $H_3BO_3$, 0.10. The trace metals solution was acidified with concentrated HCl to dissolve the salts. The strains were grown at 55°C for 24-40 hours (initial pH 7.0). The CGTases are produced extracellularly and were recovered from the cell-free broth using methods known to the art.

*C. thermoamylolyticum* ATCC 39,252 and *C. thermohydrosulfuricum* ATCC 53,016 both produced levels of CGTase sufficient for characterization.

Cyclodextrin glycosyl transferase assay

The CGTase starch-dextrinizing activity was measured by the Pharmacia Phadebase® Amylase Test at pH 6.0, 60°C by incubating 200 μl of the enzyme solution with 4.0 ml 0.1M sodium acetate (100 ppm $Ca^{++}$) plus a Phadebas Tablet for 15 minutes. The reaction was then stopped with 0.5 ml 1.0 N HCl. The assay solution was centrifuged for 2 minutes in an Eppendorf centrifuge at room temperature, and the absorbance of the supernatant was read at 620 nm where an absorbance value of 1.0-3.0 should be achieved. A standard curve using *Bacillus licheniformis* alpha-amylase as the standard was constructed where one Phadebas unit is defined as the amount of enzyme that will catalyze the hydrolysis of 1.0 μmole of glucosidic linkages of Lintner starch per minute at 60°C, pH 6.0.

Characterization of *Clostridia* CGTases

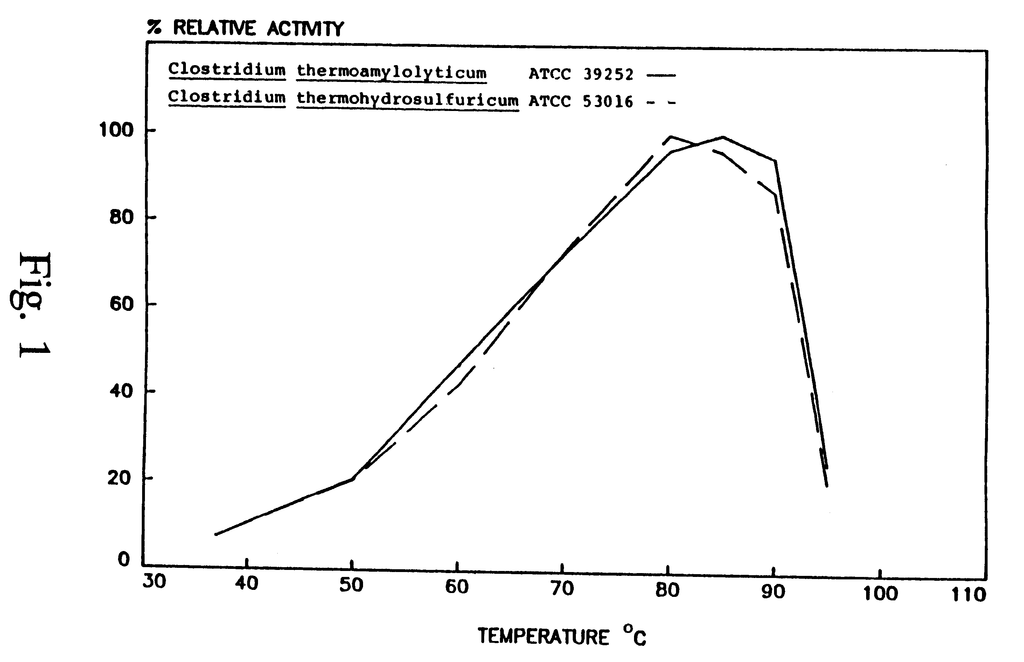

**Temperature optimum.** The effect of temperature on the CGTase activities was determined at pH 5.0 in 0.1M sodium acetate (100 ppm $Ca^{++}$). The *C. thermoamylolyticum* and *C. thermohydrosulfuricum* CGTases possess temperature optimum of 85°C and 80°C, at pH 5.0, respectively (see Figure 1).

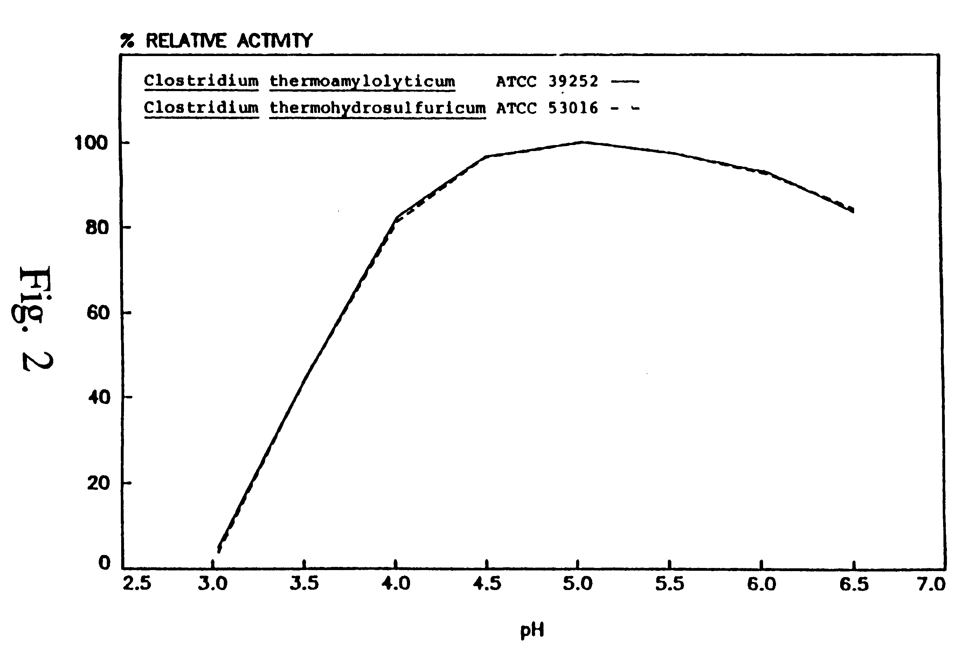

**pH optimum.** The effect of pH on the CGTase activities was examined at 60°C in a citrate-phosphate-0.5% Lintner starch (100 ppm $Ca^{++}$) buffer system. The pH optimum of both CGTases is 5.0 (see Figure 2).

**Molecular weights.** The molecular weights of the CGTases were determined by SDS-polyacrylamide gel electrophoresis to be 110 kD for the CGTases from both *C. thermoamylolyticum* and *C. thermohydrosulfuricum*.

**Isoelectric points.** Isoelectric focusing employing a LKB Ampholine PAG plate pH 3.5-9.5 followed by a 0.8% Lintner starch-iodine overlay at pH 6.0, 55°C demonstrated that the isoelectric points for both CGTases are 4.8.

There are several notable differences between the *Clostridium* CGTases and the prior-art *Thermoanaerobacter* CGTases:

(1) The temperature optimum for the *Clostridium* CGTases range from 80-85°C relative to 95°C for the *Thermoanaerobacter* CGTase.

(2) The isoelectric point for both *Clostridium* CGTases is 4.8 which compares to approximately 4.5 for the Thermoanaerobacter CGTase. The molecular weights based on SDS-PAGE are the same.

(3) The *Clostridium* CGTases are unable to liquefy solid starch at pH 4.5 unlike the *Thermoanaerobacter* CGTase.

(4) The *Clostridium* CGTases produce cyclodextrins more favorably from starch that has been partially hydrolyzed to a DE of approximately 7.0. The *Thermoanaerobacter* CGTase prefers unhydrolyzed starch.

Quantitation of cyclodextrin product

The yields of α-, β-, and γ-cyclodextrins were determined by Bio-Rad Aminex® Carbohydrate HPX-42A High Performance Liquid Chromatography. Two columns (300 x 7.8mm) were used in tandem at 85°C with glass distilled water as the eluent at a flow rate of 0.6 ml/minute. Detection was by refractive index. Standard curves were constructed with authentic samples of α-, β-, and γ-cyclodextrins (Sigma Chemical Company, St. Louis, Missouri).

**Liquefying activity.** The starch liquefying activity of the CGTases was determined under simulated industrial conditions. The CGTases were incubated with 35% DS corn starch at either pH 4.5, 5.0, 5.5, or 6.0 for 14 minutes at 105°C followed by 4 hours at 90°C. The enzyme dose was 4.46 Phadebas units per gram DS starch. The starch was judged as liquefied if it was pourable. Also, dextrose equivalents (DE) were measured by the neocuproine method.

The results (see Table 1) demonstrated that both CGTases are able to liquefy corn starch at pHs 5.5 and 6.0 while only the *C. thermohydrosulfuricum* CGTase can liquefy at pH 5.0. The DE measurements were less than 1.0 which is consistent with CGTase.

## TABLE I

### Liquefying activity

| pH | 40 ppm $Ca^{++}$ | Liquefied | |
| --- | --- | --- | --- |
| | | *Clostridium thermoamylolyticum* | *Clostridium thermohydrosulfuricum* |
| 6.0 | + | yes | yes |
| 5.5 | + | yes | yes |
| 5.0 | + | no | yes |
| 4.5 | + | no | no |
| 4.5 | + | no | no |

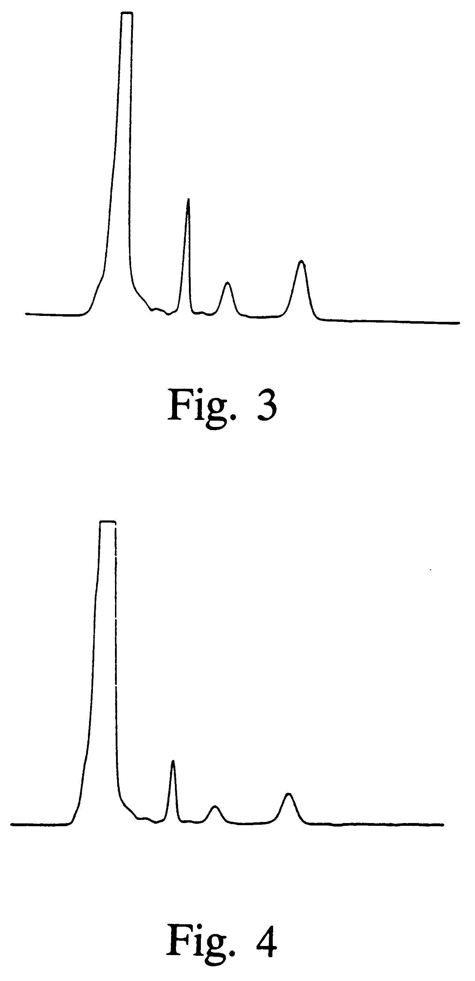

**Action patterns.** The action patterns of the liquefied starches produced by the two *Clostridium* CGTases as determined by Bio-Rad Aminex® Carbohydrate HPX-42A HPLC were characteristic of a CGTase (see Figures 3 and 4).

## DETAILED PRACTICE OF THE INVENTION

The following exemplary material is presented to more fully understand the process of the invention.

## EXAMPLE 1

Cyclodextrin production

The ability of the CGTases to produce cyclodextrins was determined at 25% DS. The slurries were liquefied by a 14 minute treatment at 105°C followed by a 4 hour hold at 90°C using an enzyme dose of 4.46 Phadebas unit per gram DS starch in the presence of 40 ppm $Ca^{++}$. The production of cyclodextrin was achieved by incubating the solution an additional 24 hours at 90°C. Liquefaction and cyclodextrinization were performed at pH 5.5 with the *C. thermoamylolyticum* CGTase and at pH 5.0 with the *C. thermohydrolsulfuricum* CGTase.

Both *Clostridia* CGTases were primarily β-cyclodextrin producers with 25% DS corn starch, but the *C. thermoamylolyticum* CGTase was more effective in producing cyclodextrins than the *C. thermohydrosulfuricum* CGTase (see Table II).

## TABLE II

| CGTase | % DS | % yield | Yield g/100 ml | | | |
|--------|------|---------|-------|------|-------|-------|
| | | | Alpha | Beta | Gamma | Total |
| C. thermoamylolyticum | 25 | 16.8 | 1.09 | 2.00 | 1.10 | 4.19 |
| C. thermohydrosulfuricum | 25 | 8.4 | 0.74 | 0.91 | 0.44 | 2.09 |

**EXAMPLE 2**

Effect of starch pretreatment on cyclodextrin yield

The effect of starch pretreatment on cyclodextrin yield was examined using Maltodextrin M50 and Maltodextrin M100 at concentrations of 25% DS. The production of cyclodextrin was accomplished by incubating the maltodextrin solutions for 24 hours at 90°C using an enzyme dose of 4.46 Phadebas units per gram DS maltodextrin in the presence of 40 ppm $Ca^{++}$. Cyclodextrinization was performed at pH 5.5 with the C. thermoamylolyticum CGTase and at pH 5.0 with the C. thermohydrosulfuricum CGTase.

The results (see Table III) demonstrate that by using preliquefied starch, i.e., Maltodextrin M50, the cyclodextrin yields produced by the C. thermohydrosulfuricum CGTase can be substantially increased. In both cases, as the DE increases the total cyclodextrin yield decreases.

## TABLE III

### Effect of starch pretreatment on cyclodextrinization

| CGTase | Malto-dextrin | Treat-ment | DE | % yield | Yield g/100 ml | | | |
|--------|---------------|------------|-----|---------|-------|------|-------|-------|
| | | | | | Alpha | Beta | Gamma | Total |
| C. thermo-amylolyticum | M50 | Acid | 7.0 | 18.0 | 1.20 | 2.36 | 0.94 | 4.50 |
| | M100 | Acid/ Enzyme | 12.0 | 9.2 | 0.51 | 1.56 | 0.23 | 2.30 |
| C. thermohydro-sulfuricum | M50 | Acid | 7.0 | 17.8 | 1.16 | 2.51 | 0.80 | 4.47 |
| | M100 | Acid/- Enzyme | 12.0 | 8.8 | 0.50 | 1.53 | 0.16 | 2.19 |

## EXAMPLE 3

The effect of reaction temperature

The enzyme preparations from *C. thermoamylolyticum* ATCC 39,252 and *C. thermohydrosulfuricum* ATCC 53,016 were employed to treat a 20% DS, 90 fluidity corn starch at pH 4.5 (American Maize, Hammond, Indiana), 70°C for 24 hours in the presence of 200 ppm Ca$^{++}$. The enzyme dose was 100 Phadebas units/g DS. The action patterns were determined by Bio-Rad Aminex® HPX-42A HPLC. Dextrose equivalents (DE) were measured by the neocuproine method.

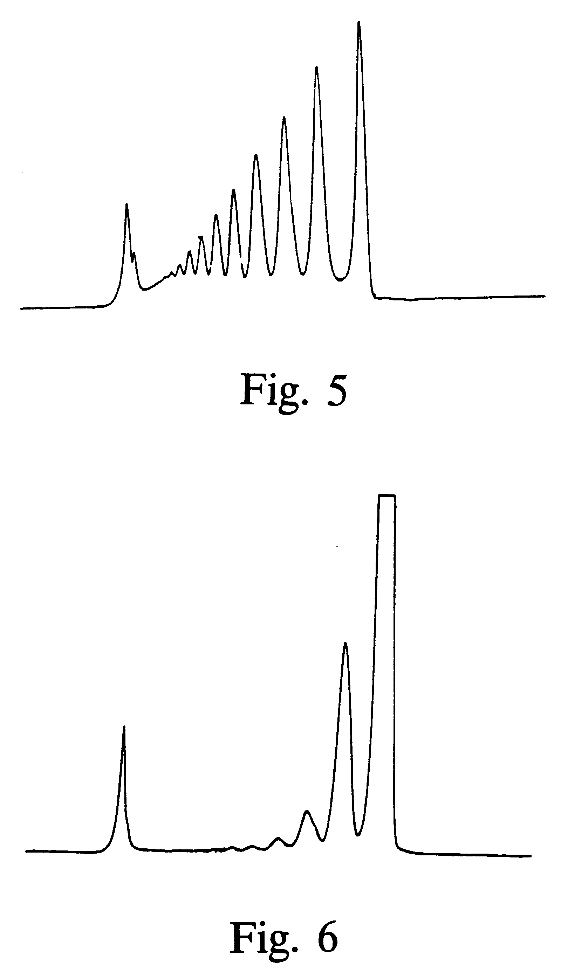

The action patterns as illustrated in Figures 5 and 6 clearly suggest the presence of a glucoamylase-like activity in each preparation. This is further confirmed by the DE measurements.

The action patterns and DE (see Figures 5 and 6) are consistent with the data reported by US 4,578,352 at the same pH, conversion temperature, and DS levels. Little or no cyclodextrin product is to be found in the 70°C hydrolysate. The temperature of 90°C employed in Examples 1 and 2 inactivates any glucoamylase activity in each preparation.

## Claims

1. A process for producing cyclodextrin comprising enzymatic treatment at 70-90°C, pH 5.0-5.5 of a liquefied starch solution with cyclodextrin glycosyl transferase (CGTase) derived from a strain of *Clostridium* and thereafter recovery of a cyclodextrin product from the reaction mixture.

2. The process of Claim 1 wherein said enzymatic treatment is conducted at a reaction time not more than about 24 hours.

3. The process of Claim 1 or 2 wherein said liquefied starch is generated by enzymatic liquefaction of a starch slurry with said cyclodextrin glycosyl transferase.

4. The process of any preceding claim wherein said enzymatic treatment of liquefied starch is conducted at a temperature of at least about 85°C.

5. A process of any preceding claim, wherein *Clostridium thermoamylolyticum* (preferably strain ATCC 39,252) or *Clostridium thermohydrosulfuricum* (preferably strain ATCC 53,016) is the source microorganism for the cyclodextrin glycosyl transferase.

6. A process for producing cyclodextrin by enzymatic treatment above 100°C at pH 5.0-5.5 of a starch slurry with a cyclodextrin glycosyl transferase (CGTase) derived from a strain of *Clostridium*, and thereafter enzymatically converting the so liquefied starch into cyclodextrin at 70-90°C, pH 5.0-5.5 with the same CGTase and then recovering a cyclodextrin product from the reaction mixture.

7. The process of Claim 6, wherein *Clostridium thermoamylolyticum* (preferably strain ATCC 39,252) or *Clostridium thermohydrosulfuricum* (preferably strain ATCC 53,016) is the source microorganism for the cyclodextrin glycosyl transferase.

## Patentansprüche

1. Verfahren zur Herstellung von Cyclodextrin, welches enzymatische Behandlung bei 70-90°C, pH 5,0-5,5, einer verflüssigten Stärkelösung mit Cyclodextrin-Glykosyltransferase (CGTase), gewonnen aus einem Clostridium-Stamm, und danach Gewinnung eines Cyclodextrin-Produktes aus der Reaktionsmischung umfaßt.

2. Verfahren nach Anspruch 1, wobei besagte enzymatische Behandlung bei einer Reaktionszeit von nicht mehr als etwa 24 Stunden durchgeführt wird.

3. Verfahren nach Anspruch 1 oder 2, wobei besagte verflüssigte Stärke durch enzymatische Verflüssigung einer Stärkeaufschlämmung mit besagter Cyclodextrin-Glykosyltransferase erzeugt wird.

**EP 0 506 790 B1**

4. Verfahren nach einem der vorangehenden Ansprüche, wobei besagte enzymatische Behandlung von verflüssigter Stärke bei einer Temperatur von wenigstens etwa 85°C durchgeführt wird.

5. Verfahren nach einem der vorangehenden Ansprüche, wobei Clostridium thermoamylolyticum (vorzugsweise Stamm ATCC 39,252) oder Clostridium thermohydrosulfuricum (vorzugsweise Stamm ATCC 53,016) der Quellen-Mikroorganismus für die Cyclodextrin-Glykosyltransferase ist.

6. Verfahren zur Herstellung von Cyclodextrin durch enzymatische Behandlung oberhalb 100°C bei pH 5,0-5,5 einer Stärkeaufschlämmung mit einer Cyclodextrin-Glykosyltransferase (CGTase), gewonnen aus einem Clostridium-Stamm, und danach enzymatische Umwandlung der so verflüssigten Stärke in Cyclodextrin bei 70-90°C, pH 5,0-5,5, mit derselben CGTase und dann Gewinnung eines Cyclodextrin-Produktes aus der Reaktionsmischung.

7. Verfahren nach Anspruch 6, wobei Clostridium thermoamylolyticum (vorzugsweise Stamm ATCC 39,252) oder Clostridium thermohydrosulfuricum (vorzugsweise Stamm ATCC 53,016) der Quellen-Mikroorganismus für die Cyclodextrin-Glykosyltransferase ist.


**Revendications**

1. Procédé de production de cyclodextrine comprenant un traitement enzymatique à 70-90°C, à un pH de 5,0-5,5, d'une solution d'amidon liquéfié avec de la cyclodextrine glycosyltransférase (CGTase) dérivée d'une souche de *Clostridium*, puis la récupération de la cyclodextrine produite à partir du mélange réactionnel.

2. Procédé selon la revendication 1 dans lequel ledit traitement enzymatique est effectué pendant un temps de réaction ne dépassant pas 24 heures environ.

3. Procédé selon la revendication 1 ou 2 dans lequel ledit amidon liquéfié est produit par liquéfaction enzymatique d'une pâte d'amidon avec ladite cyclodextrine glycosyltransférase.

4. Procédé selon l'une quelconque des revendications précédentes, dans lequel ledit traitement enzymatique de l'amidon liquéfié est effectué à une température d'au moins 85°C environ.

5. Procédé selon l'une quelconque des revendications précédentes, dans lequel *Clostridium thermoamylolyticum* (de préférence la souche ATCC 39 252) ou *Clostridium thermohydrosulfuricum* (de préférence la souche ATCC 53 016) est le microorganisme producteur de la cyclodextrine glycosyltransférase.

6. Procédé de production de cyclodextrine par traitement enzymatique au-dessus de 100°C, à un pH de 5,0-5,5, d'une pâte d'amidon avec une cyclodextrine glycosyltransférase (CGTase) dérivée d'une souche de *Clostridium*, puis conversion enzymatique de l'amidon ainsi liquéfié en cyclodextrine à 70-90°C, à un pH de 5,0-5,5, avec la même CGTase et récupération de la cyclodextrine produite à partir du mélange réactionnel.

7. Procédé selon la revendication 6, dans lequel *Clostridium thermoamylolyticum* (de préférence la souche ATCC 39 252) ou *Clostridium thermohydrosulfuricum* (de préférence la souche ATCC 53 016) est le microorganisme producteur de la cyclodextrine glycosyltransférase.

8

Fig. 1

% RELATIVE ACTIVITY

Clostridium thermoamylolyticum ATCC 39252 ——

Clostridium thermohydrosulfuricum ATCC 53016 – –

Fig. 2

pH

EP 0 506 790 B1

Fig. 3

Fig. 4

Fig. 5

Fig. 6